# EUROPÄISCHE PATENTSCHRIFT

(11) **EP 1 762 204 B1**
(45) Veröffentlichungstag und Bekanntmachung des Hinweises auf die Patenterteilung: **13.10.2010**
(21) Anmeldenummer: 06016290.6
(22) Anmeldetag: 04.08.2006
(51) Int. Cl.: A61F 13/00, A61F 13/534

(54) **Vliesmaterial, Verbandmaterial, Verfahren zum Herstellen eines Verbandmaterials**
Nonwoven, bandage material, method of producing a bandage material
Non-tissé, matériau à pansement, procédé pour produire un materiau à pansement

(30) Priorität: 13.09.2005 DE 102005045655
(43) Veröffentlichungstag der Anmeldung: 14.03.2007
(73) Patentinhaber: Paul Hartmann AG, 89522 Heidenheim (DE)
(72) Erfinder: Malowaniec, Krzysztof D., 89522 Heidenheim (DE)
(74) Vertreter: Dreiss

(56) Entgegenhaltungen:
- EP-A- 0 858 790
- DD-A1- 239 013

## Beschreibung

Die Erfindung betrifft ein Verbundmaterial bestehend aus einem medizinisches Vliesmaterial gemäß Anspruch 1 sowie ein Verfahren zu dessen Herstellung gemäß Anspruch 7. Derartige Vliesmaterialien sind bekannt. So offenbart beispielsweise die DE 197 05 737 A1 ein Verfahren zur Herstellung medizinischer Saugkörper, wobei als Rohstoff ein Baumwollfasermaterial eingesetzt wird. Zur Herstellung des Saugkörpers wird das Fasermaterial im ersten Schritt zu einer ersten und einer zweiten Deckschicht vermascht und im zweiten Schritt werden die Fasern zwischen der ersten und der zweiten Deckschicht unter Bildung von Zwischenräumen zu der ersten und zweiten Deckschicht senkrecht ausgerichtet.

Durch das Vermaschen wird u. a. erreicht, dass die zunächst losen Fasern, insbesondere Naturfasern, aus denen entsprechende Vliesmaterialien hergestellt sind, zusammengehalten und so die Einzelfasern am Auseinanderdriften gehindert werden. Auf diese Weise wird die Flüssigkeitsaufnahme und das Speicherverhalten ermöglicht, welches ein Mehrfaches des Eigengewichts des Vliesmaterials ausmacht. Darüber hinaus lassen sich dann die Vliesmaterialien, sofern sie beispielsweise als Verbandmaterialien dienen, im Gesamten von einer Wunde entfernen und damit auch die von ihnen aufgesogene Flüssigkeit. Es ist dabei bei Verbandmaterialien, aber auch bei kosmetischen Materialien, als negativ anzusehen, wenn viele Fasern aus dem Gesamtverbund gelöst werden und gegebenenfalls im oder am Körper verbleiben und so zu Abwehrreaktionen oder einem unwohlen Gefühl führen können.

Mit den Schriften DE 198 04 940 A1 sowie DE 198 39 418 C1 und DE 198 43 078 A1 werden weitere Verfahren zur Vermaschung einer Oberseite eines Vliesmaterials beschrieben, nämlich die sogenannten Kunit-, Maliknitsowie das Multiknit-Verfahren.

Sofern Vliesmaterialien zur Herstellung von medizinischen Kompressen eingesetzt werden sollen, müssen die Auflagen der DIN EN 14079 erfüllt werden.

Es ist daher Aufgabe der Erfindung, ein alternatives Vliesmaterial bereitzustellen, das insbesondere in der Herstellung von medizinischen Kompressen Einsatz finden kann.

Die Erfindung löst diese Aufgabe durch ein medizinisches Vliesmaterial umfassend ein Flachmaterial, dessen beide Oberseiten zur Bildung eines Vlieswirkstoffs, d.h. eines Vlieses mit einer gewirkten Oberfläche, vermascht sind, und wobei das Vlieswirkstoffmaterial gebleicht ist. Hierbei wird das bereits fertig vermaschte Vlieswirkstoffmaterial einem Bleichprozess unterzogen.

Überraschender Weise hat sich herausgestellt, dass ein Vlieswirkstoff mit einer vermaschten Oberseite in einem der Vliesbildung nachfolgenden Bleichprozess keine Nachteile hinsichtlich seiner Festigkeit und Integrität erfährt. Üblicherweise wird in einem solchen Bleichprozess, insbesondere bei der Verwendung unselektiver umweltfreundlicher Bleichchemikalien, eine Faser derart geschädigt, dass ein z.B. im Spunlace-Verfahren verfestigter Vliesstoff keinen genügenden Zusammenhalt mehr aufweist, um beispielsweise als medizinische Kompressen eingesetzt zu werden. Offenbar wird durch die Vermaschung verhindert, dass der Zusammenhalt des Vliesstoffs beim Bleichprozess, der in der Regel ein Rotationsbleichprozess ist, zerstört wird. Des Weiteren könnte der Bleichprozess die Gefahr eines erhöhten Lintings, also Fusselns des Materials, bedingen. Überraschenderweise weisen die erfindungsgemäßen Vliesmaterialien jedoch kein erhöhtes Linting auf.

Darüber hinaus besitzt das erfindungsgemäße Vliesmaterial den Vorteil, dass das Vliesmaterial durch den nachfolgenden Bleichprozess, der vorzugsweise einen Beuchschritt enthält, weiter stabilisiert wird, im Gegensatz zum Vliesmaterial gemäß dem Stand der Technik, der z. B. vorgebleichte Baumwolle einsetzt. Auf diese Weise kann durch die Erfindung ein Vliesmaterial insbesondere zur Herstellung von Verbandmaterialien bereitgestellt werden, das eine höhere Reinheit und gleichzeitig eine höhere Festigkeit als herkömmliche Vliesmaterialien aufweist. Hierbei wird durch das Nachbehandeln, nämlich das Bleichen, nicht nur eine höhere Festigkeit als bei einem beispielsweise auf herkömmliche Art wasserstrahlverfestigten Vliesmaterial erreicht, sondern auch eine höhere Festigkeit gegenüber einem entsprechenden ungebleichten Vlieswirkstoffmaterial oder einem Material aus bereits gebleichten Fasern, das ebenfalls eine vermaschte Oberfläche aufweist. Der nachgeschaltete Bleichprozess ruft offensichtlich eine Schrumpfung der einzelnen Fasern hervor, die die Stabilität des fertigen Vlieswirkstoffes erhöht.

Durch den Bleichprozess wird der Vliesstoff derart verfestigt, das er nach dem Bleichen ein Flächengewicht von 100 bis 800 g/ m² insbesondere 100 bis 600 g/ m² und ganz besonders bevorzugt 100 bis 500 g/ m² aufweist.

Des weiteren wird der Vliesstoff durch den Bleichprozess derart verfestigt, das er nach dem Bleichen ein Dichte von 0,05 bis 0,15 g/ cm³ insbesondere 0,06 bis 0,11 g/ cm³ und ganz besonders bevorzugt 0,07 bis 0,10 g/ cm³ aufweist (Dicke gemessen bei einem Prüfdruck von 10 cN/ cm²).

Beide Oberseiten des Flachmaterials sind vermascht. Dabei können sowohl das Kunit-, als auch das Multiknit- als auch andere Vermaschverfahren, die im Stand der Technik bekannt sind, eingesetzt werden. Es können auch für die beiden Oberseiten unterschiedliche Verfahren verwandt werden.

Dabei kann es insbesondere vorgesehen sein, dass zwischen den Oberseiten die Fasern ausgerichtet sind unter Bildung von Hohlräumen. Die Hohlräume erstrecken sich jedoch nicht bis in die vermaschten Oberseiten, die eine durchgehende kontinuierliche Struktur besitzen. Insbesondere ist es weiterhin vorteilhaft, wenn die Fasern zwischen den Oberseiten des Flachmaterials unter Bildung von Hohlräumen im Wesentlichen senkrecht zu den Oberflächen ausgerichtet sind. Durch diese Hohlraumbildung und Ausrichtung der Fasern wird ein Vliesmaterial erhalten, das besonders gute Saugeigenschaften aufweist. Überraschender Weise hat es sich auch hinsichtlich dieser Hohlräume gezeigt, dass diese nicht in einem der Vliesbildung nachfolgendem Bleichprozess zerstört werden und die Saugeigenschaften des gebleichten Vlieswirkstoffs nicht beeinträchtigt wird.

Weiterhin besteht das Vliesmaterial aus Baumwollfasern oder umfaßt diese zumindest in weiten Teilen. Alternativ können auch Baumwollfasergemische mit anderen Fasern eingesetzt werden. In solchen Fasergemischen können insbesondere natürliche Fasern mit Baumwollfasern gemischt vorliegen. Als natürliche Fasern kommen hierbei insbesondere Hanf-, Sisal-, Jute-, Cocos- oder Flachsfasern zur Anwendung. Darüber hinaus können in solchen Fasergemischen jedoch auch synthetische Fasern eingesetzt werden. Als synthetische Fasern können insbesondere Polyamid-, Polyester-, Polyethylen-, Polypropylen- oder Viskosefasern eingesetzt werden. Die Baumwollfasern besitzen jedoch in ihrem Urzustand, in dem sie nicht gebleicht oder lediglich teilgebleicht sind und indem sie zum Vlieswirkstoff verarbeitet werden, einen bestehenden Restanteil an Fetten und Wachsen, wobei diese Fette und Wachse förderlich für die Bildung des Vlieswirkstoffs sind. Die Herstellung der vermaschten Oberseiten, aber auch eine Ausrichtung der Fasern im Bereich zwischen den Oberseiten wird so deutlich erleichtert. Falls Fasergemische aus Baumwollfasern und anderen natürlichen Fasern eingesetzt werden sollen, so können alle Fasern nicht gebleicht oder lediglich teilgebleicht vorliegen.

Es kann weiterhin vorgesehen sein, dass dem Herstellungsverfahren weitere Textilveredelungsschritte nachgeschaltet sind, denen das Vliesmaterial unterzogen wird. Denkbar sind insbesondere Hydrophobisierungs- sowie Hydrophilisierungsverfahren und alternativ oder ergänzend kann auch vorgesehen sein, dass ein Sterilisierprozess für herzustellende Verbandmaterialien insbesondere nach der Konfektionierung zu Verbandmaterialien, wie insbesondere Kompressen, Tamponaden etc., erfolgt. Schließlich ist es auch denkbar, Wirkstoffe in die Verbandmaterialien einzugliedern, indem diese z. B. an die Fasern angeheftet werden. Dies kann insbesondere ein antimikrobieller Wirkstoff sein. Als antimikrobieller Wirkstoff kann hierbei insbesondere Silber oder ein Silbersalz wie Silberchlorid verwendet werden.

Es ist dabei besonders vorteilhaft, wenn das Herstellungsverfahren wie folgt abläuft:

Zunächst wird ein Vliesmaterial aus ungebleichten oder teilgebleichten Baumwollfasern in herkömmlicher Weise gebildet, wobei anschließend eine oder beide Oberflächen des Flachmaterials vermascht werden und insbesondere die Fasern zwischen den Oberflächen einem Ausrichtungsprozess unterzogen werden. Die Saugfähigkeit eines Saugkörpers ist dann maximal, wenn alle Faserspitzen gleich hoch und parallel ausgerichtet sind. Speichervolumen und
Haltevermögen hängen dagegen vom sogenannten Kapillareffekt ab, d. h. von der Entfernung parallel liegender Fasern untereinander, der Affinität der Fasern zum Sekret und dessen Konsistenz. Die Fähigkeit der Fasern, Flüssigkeit zu binden, sinkt, je mehr und je größer die Faseroberfläche durch Verbundstellen mit anderen Fasern belegt ist. Jede Entwicklung eines optimalen Saugkörpers muss daher die zum Teil sich widersprechenden Anforderungen, nämlich hohe Fasereinbindung und hohes Flüssigkeits- und Haltevermögen, optimal kombinieren. Durch die Ausrichtung der Fasern im Mittelbereich und die Einbindung in ein Fasergebilde lediglich im Bereich der Deckschicht wird dies optimal gelöst. Der Hauptteil der Fasern verbleibt hierbei senkrecht und parallel zueinander angeordnet und steht damit in seiner Funktion, Flüssigkeit zu halten und zu transportieren, voll zur Verfügung. Durch die auf der anderen Seite fest vermaschte Deckschicht wird erreicht, dass das Herauslösen einzelner Fasern und die hiermit verbundene Problematik gleichzeitig reduziert ist.

Insbesondere ist in dem erfindungsgemäßen Verfahren vorgesehen, dass das Vlieswirkstoffmaterial verfestigt wird und besonders bevorzugt, durch das Bleichverfahren verfestigt wird, das heißt, dass mit dem Bleichverfahren eine weitere Verfestigung des Vlieswirkstoffs erfolgt. Weiterhin bevorzugt ist, dass das Flächengewicht des Vlieswirkstoffs erhöht wird und besonders bevorzugt, dass das Flächengewicht durch das Bleichverfahren erhöht wird, das heißt, dass eine Zunahme des Flächengewichtes des Vlieswirkstoffs während des Bleichens erfolgt. Darüber hinaus ist insbesondere auf vorgesehen, dass die Dichte des Vlieswirkstoffs erhöht wird und besonders bevorzugt, dass die Dichte durch das Bleichverfahren erhöht wird, das heißt, dass eine Zunahme der Dichte des Vlieswirkstoffs während des Bleichens erfolgt. Das Bleichverfahren soll nachstehend näher erläutert werden.

Das verwendete Bleichverfahren ist vorzugsweise ein Rotationsbleichverfahren, das weiterhin vorzugsweise ein Beuchverfahren, das dem eigentlichen Bleichen vorgeschaltet ist, umfasst. Es kann hierbei des Weiteren vorgesehen sein, dass der Vliesstoff nach dem Bleichen weiter veredelt wird. Hierbei kann insbesondere eine Konfektionierung des Materials vorgesehen sein, wobei dieses z. B. zu Kompressen gestanzt wird und eine Randverfestigung des Materials mittels Wasserstrahlvernadelung nach dem Bleichprozess erfolgt. Auf diese Weise kann erreicht werden, dass ein vollständig abgeschlossenes Gebilde in Form eines Saugkörpers zur Verfügung steht, aus dem nur geringfügig Fasern herausgelöst werden können, die, was negativ zu beurteilen ist, in einer Wunde verbleiben könnten. Die Kompressen werden dann vorzugsweise sterilisiert und einzelverpackt. Grundsätzlich ist jedoch auch die Verwendung des Materials für unsterilisierte Verbandmaterialien denkbar. Das Material besitzt darüber hinaus den Vorteil, eine hohe taktile Gefälligkeit zu besitzen, die es besonders angenehm und hautfreundlich macht.

Die Erfindung soll nun insoweit näher erläutert werden, als die Weiterbehandlung im Bleichprozess des fertigen Vlieswirkstoffmaterials dargestellt werden soll.

Das Bleichverfahren läuft dabei in folgenden Schritten ab. So wird zunächst das zu bleichende Vlieswirkstoffmaterial auf einen Metallzylinder, der eine Porosität aufweist, aufgedockt, d. h. nass aufgewickelt. Hierzu wird zuvor das zu bleichende Material durch eine Aufdockflotte gezogen und dann nass auf den Zylinder aufgerollt. Als Aufdockflotte kommt hierbei folgende Rezeptur zum Einsatz:
a) Cottoclarin OK - Fa. Cognis Deutschland GmbH & Co KG, Düsseldorf (Deutschland) 2 g/l
b) Kollasol SD - Fa. Beitlich R. GmbH & Co.KG, Tübingen (Deutschland) 2 g/l
c) 50 %-ige Natronlauge 10,5 ml/l
d) Heptol LCP - Fa. Beitlich R. GmbH & Co.KG, Tübingen (Deutschland) 3 g/l
e) K-Persulfat - Herkommer & Bangerter GmbH & Co, Ulm, Deutschland 5 g/l.

Bei dem verwendeten K-Persulfat (K₂S₂O₈) handelt es sich um ein alkalisches Bleichmittel und bei Heptol um ein Komplexierungsmittel sowie bei Kollasol um ein Netz- bzw. Schaumdämpfungsmittel und bei Cottoclarin um ein Netzmittel.

Der Zylinder wird dann in die Bleichanlage eingebracht, wobei es sich hier um eine Rotowa-Anlage (Rotowa AV41, Fa. Jaeggli Maschinenfabrik, Winterthur - Schweiz) handelt. Sämtliche Rezepturen sind dabei in Tabelle 1 dargestellt.

Als erster Schritt wird ein Beuchprozess durchgeführt, wobei während des Beuchens unter Rotation der Zylinder in der Bleichanlage bewegt wird. Das Beuchen findet unter Verwendung der sogenannten Abkochflotte statt, die eine folgende Rezeptur besitzt:
a) Cottoclarin OK 4,5 g/l
b) Heptol LCP 0,75 g/l
c) 50 %-ige Natronlauge 45 ml/l.

Mittels des Abkochens, bei dem die Temperatur von 40° auf 98° erhöht wird und die Drehzahl zwischen 20 und 50 Umdrehungen pro Minute schwankt, erfolgt ein Verseifen der Fettsäureester in der Baumwolle, die so wasserlöslich gemacht werden.

Im nächsten Schritt erfolgt nun ein Spülen mit klarem Wasser, wobei hieran der eigentliche Bleichschritt anschließt, bei dem wiederum eine Kreislaufbewegung des Zylinders stattfindet. Hierzu wird die Bleichflotte bestehend aus:
a) Magnesiumsulfat 0,200 g/l
b) Contavan TIG - Fa. Beitlich R. GmbH & Co.KG, Tübingen (Deutschland) 3 g/l
c) 50 %-ige Natronlauge 9,5 ml/l
d) Wasserstoffperoxid (50 %ig in Wasser) 33 ml/l
   als Peroxidbleiche eingesetzt. Auch während des Bleichens wird die Temperatur von 40° auf 93° erhöht. Die Umdrehungszahl der Trommel liegt zwischen 20 und 50 Umdrehungen pro Minute. Das in der eigentlichen Bleichstufe eingesetzte Contavan wird zur Stabilisierung des Peroxids eingesetzt, wobei es zu einem gezielten Zerfall und Freisetzung des aktiven Sauerstoffs kommt. An den eigentlichen Bleichprozess schließt sich ein weiterer Spülprozess an, bei dem die Temperatur bei 98° C verbleibt.

In einer dritten Stufe erfolgt nun eine Nachbehandlung mit dem Komplexiermittel Periquest SD (Textilchemie Dr. Petry GmbH, Reutlingen - Deutschland), das in einer Menge von 6,5 g/l eingesetzt wird, wobei wiederum eine Rotation des Zylinders stattfindet. Durch die Zugabe von Periquest werden die noch vorhandenen Metallionen aus dem Material entfernt. Wiederum nachgeschaltet ist nun eine Spülstufe, die bei Temperaturen zwischen 10° und 98° stattfindet. Hieran anschließend wird eine weitgehende Neutralisation der vorher alkalischen Flüssigkeit mit 60%-iger Essigsäure durchgeführt, um einen pH-Wert zwischen 4 und 6 einzustellen. Zum Abschluss erfolgt ein Schleuderprozess. Der gesamte Bleichprozess besitzt eine Prozessdauer von 240 min und ist in Tabelle 2 abgebildet. Dabei wird Wasser der Härte 0 bis 1 eingesetzt. Das vollständige Trocknen des gebleichten Vlieswirkstoffs wird außerhalb der Rotowa-Anlage durchgeführt.

In den nachfolgenden Tabellen sind zum einen die eingesetzten Rezepturen in Tabelle 1 angegeben und zum anderen ist der Bleichprozess in der Rotowa-Anlage in Tabelle 2, wie er vorstehend bereits erläutert wurde, mit den jeweiligen Temperaturen sowie den Füllständen in der Anlage und den Durchflüssen in m³/h dargestellt.

Durch das vorstehend beschriebene Verfahren wird erreicht, dass ein Verbandmaterial von einer Qualität hergestellt werden kann, wie sie der vorliegenden DIN-Norm EN 14079 entspricht und auf der anderen Seite eine hohe Saugfähigkeit und hohe Festigkeit bei gleichzeitig geringem Faserverlust erzielt werden kann.

### Tabelle 1 - REZEPTUR

**Beim Flottenansatz ist die Reihenfolge der Komponenten einzuhalten**

| FLOTTENANSATZ für | | 1.000 l | 2.000 l | 3.000 l | 4.000 l |
|---|---|---|---|---|---|
| Aufdocken | | | | | |
| (Flottenverhältnis ca. 1:1,5; 1:1,75) | | | | | |
| Cottoclarin OK (Netzmittel) | 2 g/l | 2,00 kg | 4,00 kg | 6,00 kg | 8,00 kg |
| Kollasol SD | 2 g/l | 2,00 kg | 4,00 kg | 6,00 kg | 8,00 kg |
| 50 %-ige Natronlauge | 10,5 ml/l | 10,5 l | 21,0 l | 31,5 l | 42,0 l |
| Heptol LCP | 3 g/l | 3,00 kg | 6,00 kg | 9,00 kg | 12,00 kg |
| K-Persulfat (Entschlichtemittel) | 5 g/l | 5,00 kg | 10,00 kg | 15,00 kg | 20,00 kg |

| Abkochen | | | | | |
|---|---|---|---|---|---|
| Flottenverhältnis ca. 1:1 + 1:1,25) | | | | | |
| Cottoclarin OK | 4,5 g/l | 4,50 kg | 9,00 kg | 13,50 kg | 18,00 kg |
| Heptol LCP | 0,75 g/l | 0,75 kg | 1,50 kg | 2,25 kg | 3,00 kg |
| 50 %-ige Natronlauge | 45 ml/l | 45,0 l | 90,0 l | 135,0 l | 180,0 l |

| Peroxidbleiche | | | | | |
|---|---|---|---|---|---|
| (Flottenverhältnis 1:1) | | | | | |
| Magnesiumsulfat | 0,200 g/l | 0,20 kg | 0,40 kg | 0,60 kg | 0,80 kg |
| Contavan TIG | 3 g/l | 3,00 kg | 6,00 kg | 9,00 kg | 12,00 kg |
| 50 %-ige Natronlauge | 9,5 ml/l | 9,5 l | 19,0 l | 28,5 l | 38,0 l |
| Wasserstoffperoxid | | 33,0 l | 66,0 l | 99,0 l | 132,0 l |
| (50 %-ig in Wasser) | 33 ml/l | | | | |

| Nachbehandlung | | | | | |
|---|---|---|---|---|---|
| (Flottenverhältnis 1:1) | | | | | |
| Komplexiermittel Periquest SD | 6,5 g/l | 6,50 kg | 13,00 kg | 19,50 kg | 26,00 kg |

| Neutralisation | | | | | |
|---|---|---|---|---|---|
| (Flottenverhältnis 1:1) | | | | | |
| Essigsäure 60 % | | 6,0 l | 12,0 l | 18,0 l | 24,0 l |

**Tabelle 2 - Prozessschritte des Bleichens**

| Schritt Nr. | Stufe | Parameter | | | | | Behälter | |
|---|---|---|---|---|---|---|---|---|
| | | Zeit (s) | Füllstand Zufluss | Durchfluss m³/h | Temp. °C | Drehzahl U/min | von | nach |
| | Abkochen - Kreislauf | | | | | | | |
| 1 | Abkochflotte abrufen | 30 | | 0 | 40 | 20 | 5 | |
| 2 | | --- | 45% 1200 l | 20 | 40 | 20 | 5 | |
| 3 | | 120 | | 25 | 40 | 50 | | |
| 4 | | 120 | | 25 | 40 | 50 | | |
| 5 | | 300 | | 25 | 60 | 20 | | |
| 6 | | 300 | | 25 | 80 | 30 | | |
| 7 | | 300 | | 25 | 80 | 20 | | |
| 8 | | 840 | | 25 | 98 | 30 | | |
| 9 | | 900 | | 25 | 98 | 20 | | WRG |

| | 1. Spülen | | | | | | | |
|---|---|---|---|---|---|---|---|---|
| 10 | | 720 | | 20 | 98 | 20 | 3 | |
| 11 | | 240 | | 20 | 98 | 20 | 3 | WRG |
| 12 | | 240 | | 20 | 98 | 100 | 3 | WRG |
| 13 | | 240 | | 20 | 98 | 20 | 3 | WRG |
| 14 | | 300 | | 20 | 98 | 200 | 3 | WRG |
| 15 | | 240 | | 20 | 98 | 20 | 3 | WRG |
| 16 | | 240 | | 20 | 98 | 200 | 3 | WRG |
| 17 | | 300 | | 25 | 98 | 260 | 2 | 3 |
| 18 | | 300 | | 25 | 10 | 200 | 1 | 3 |
| 19 | | 120 | | 25 | 10 | 200 | 1>60°C | WRG |
| 20 | | --- | 20% | 25 | 10 | 20 | 1 | |
| 21 | | 150 | | 25 | 10 | 100 | | |

| | Bleichen - Kreislauf | | | | | | | |
|---|---|---|---|---|---|---|---|---|
| 22 | Bleichflotte abrufen | 180 | | 0 | 30 | 200 | 7 | |
| 23 | | --- | 35% 900 l | 20 | 40 | 20 | 7 | |
| 24 | | 120 | | 25 | 40 | 50 | | |
| 25 | | 300 | | 25 | 60 | 20 | | |
| 26 | | 480 | | 25 | 80 | 30 | | |
| 27 | | 300 | | 25 | 93 | 20 | | |
| 28 | | 240 | | 25 | 93 | 20 | | |
| 29 | | 180 | | 25 | 93 | 20 | | |
| 30 | | 120 | | 25 | 93 | 20 | | |
| 31 | | 60 | | 25 | 93 | 20 | | |
| 32 | | 900 | | 25 | 93 | 20 | | WRG |

| | 2. Spülen | | | | | | | |
|---|---|---|---|---|---|---|---|---|
| 33 | | 120 | | 25 | 98 | 20 | 2 | WRG |
| 34 | | 120 | | 25 | 98 | 50 | 2 | WRG |

| | Komplexieren - Kreislauf | | | | | | | |
|---|---|---|---|---|---|---|---|---|
| 35 | Periquest abrufen | 180 | | 10 | 10 | 200 | 8 | |
| 36 | | - | 35% 900 l | 20 | 80 | 20 | 8 | |
| 37 | | 120 | | 25 | 98 | 50 | | |
| 38 | | 360 | | 25 | 98 | 20 | | WRG |

| | 3. Spülen | | | | | | | |
|---|---|---|---|---|---|---|---|---|
| 39 | | 240 | | 25 | 98 | 20 | 2 | WRG |
| 40 | | 90 | | 25 | 10 | 20 | 2 | WRG |
| 41 | | -- | 35% 900 l | 25 | 98 | 20 | 2 | |
| 42 | | 240 | | 25 | 98 | 30 | | |
| 43 | | 780 | | 25 | 98 | 200 | 2 | WRG |
| 44 | | 60 | | 25 | 10 | 20 | 2 | WRG |
| 45 | | --- | 35% 900 l | 25 | 98 | 20 | 2 | |
| 46 | | 240 | | 25 | 98 | 20 | | |
| 47 | | 60 | | 25 | 98 | 30 | 2 | 3 |
| 48 | | 600 | | 25 | 98 | 260 | 2 | 3 |
| 49 | | 480 | | 25 | 40 | 200 | 1 | 3 |
| 50 | | 120 | | 25 | 10 | 20 | 1>60°C | WRG |

| | Säurestufe - Kreislauf | | | | | | | |
|---|---|---|---|---|---|---|---|---|
| 51 | Säure abrufen | 180 | | 10 | 10 | 200 | 6 | |
| 52 | | --- | 35% 900 l | 20 | 40 | 20 | 6 | |
| 53 | | 600 | | 25 | 40 | 20 | | |
| 54 | | 300 | | 25 | 40 | 100 | | |

| | Entschleudern | | | | | | | |
|---|---|---|---|---|---|---|---|---|
| 55 | | 420 | | 0 | 40 | 260 | | |
| 56 | | 120 | | 0 | 40 | 260 | | |
| 57 | | 60 | | 0 | 40 | 260 | | |
| 58 | Programmende | 30 | | 0 | 40 | 20 | | |

| | | | | | | | | |
|---|---|---|---|---|---|---|---|---|
| Insgesamt 240 Minuten Behälter 1: Wasser weich kalt Behälter 2: Wasser weich heiß (60°C - 80°C) Behälter 3: Recyclingwasser Behälter 4: WRG (60°C - 98°C) - Wärmerückgewinnung Behälter 5: Abkochflotte kalt (30°C - 40°C) Behälter 6: Säure Behälter 7: Bleichflotte kalt Behälter 8: Komplexierflotte | | | | | | | | |

## Patentansprüche

1. Verbandmaterial bestehend aus einem medizinischen Vliesmaterial, umfassend ein Flachmaterial, dessen beide Oberseiten zur Bildung eines Vlieswirkstoffs vermascht sind, wobei das Vlieswirkstoffmaterial gebleicht ist und das Vliesmaterial aus Baumwollfasern oder einem Baumwollfasergemisch besteht.

2. Verbandmaterial nach Anspruch 1, wobei das Vermaschen nach dem Kunit-Verfahren und/oder Multiknit-Verfahren erfolgt ist.

3. Verbandmaterial nach Anspruch 1 oder 2, wobei die Fasern zwischen den Oberseiten unter Bildung von Hohlräumen ausgerichtet sind.

4. Verbandmaterial nach einem der vorangehenden Ansprüche, wobei das Vlieswirkstoffmaterial mittels Textilveredelungsschritten veredelbar ist.

5. Verbandmaterial nach einem der vorangehenden Ansprüche, wobei an Fasern des Vlieswirkstoffmaterials medizinische oder kosmetische Wirkstoffe angeheftet sind.

6. Verbandmaterial nach einem der vorangehenden Ansprüche, wobei es eine Kompresse oder Tamponade ist.

7. Verfahren zum Herstellen eines medizinischen Vliesmaterials für Verbandmaterialien aus Baumwollfasern oder Baumwollfasergemisch nach einem der vorangehenden Ansprüche, wobei das Flachmaterial mittels eines Wirkverfahrens an beiden Oberflachen zur Bildung eines Vlieswirkstoffmaterials vermascht wird und wobei das Vlieswirkstoffmaterial anschließend einem Bleichverfahren unterzogen wird.

8. Verfahren nach Anspruch 7, wobei das Vlieswirkstoffmaterial verfestigt wird.

9. Verfahren nach Anspruch 7 oder 8, wobei die Dichte oder das Flächengewicht des Vlieswirkstoffmaterials erhöht wird.

10. Verfahren nach mindestens einem der Ansprüche 7 bis 9, wobei die Verfestigung oder die Erhöhung des Flächengewichtes oder die Erhöhung der Dichte des Vlieswirkstoffmaterials durch das Bleichverfahren erfolgt.

11. Verfahren mindestens einem der Ansprüche 7 bis 10, wobei das Bleichverfahren ein Rotationsbleichverfahren ist.

12. Verfahren nach einem oder mehreren der vorangehenden Ansprüche 7 bis 11, wobei das Vliesmaterial nach dem Bleichen weiter veredelt wird.

13. Verfahren nach einem oder mehreren der vorangehenden Ansprüche 7 bis 12, wobei das Material zu Kompressen gestanzt wird, wobei die Konfektionierung vor oder nach dem Bleichen erfolgt und die Kompressen randverfestigt werden, insbesondere mittels Wasserstrahlvernadelung.

14. Verfahren nach Anspruch 13, wobei die Kompressen sterilisiert und einzelverpackt werden.

15. Verfahren nach einem der vorangehenden Ansprüche, wobei die eingesetzten Baumwollfasern bei der Vliesbildung ungebleicht oder teilgebleicht sind.

## Claims

1. Bandaging material consisting of a medical nonwoven material, comprising a flat material, the two upper sides of which are meshed in order to form a nonwoven knit, wherein the nonwoven knit material is bleached and the nonwoven material consists of cotton fibres or a cotton fibre mixture.

2. Bandaging material according to Claim 1, wherein the meshing takes place by means of the Kunit process and/or Multiknit process.

3. Bandaging material according to Claim 1 or 2, wherein the fibres between the upper sides are oriented to form cavities.

4. Bandaging material according to one of the preceding claims, wherein the nonwoven knit material can be refined by means of textile refinement steps.

5. Bandaging material according to one of the preceding claims, wherein medical or cosmetic active substances are attached to fibres of the nonwoven knit material.

6. Bandaging material according to one of the preceding claims, wherein it is a compress or tampon.

7. Method for producing a medical nonwoven material for bandaging materials consisting of cotton fibres or a cotton fibre mixture according to one of the preceding claims, wherein the flat material is meshed by means of a knitting process on both upper surfaces in order to form a nonwoven knit material, and wherein the nonwoven knit material is then subjected to a bleaching process.

8. Method according to Claim 7, wherein the nonwoven knit material is consolidated.

9. Method according to Claim 7 or 8, wherein the density or the weight per unit area of the nonwoven knit material is increased.

10. Method according to at least one of Claims 7 to 9, wherein the consolidation or the increase in the weight per unit area or the increase in density of the nonwoven knit material takes place as a result of the bleaching process.

11. Method according to one of Claims 7 to 10, wherein the bleaching process is a rotary bleaching process.

12. Method according to one or more of the preceding Claims 7 to 11, wherein the nonwoven material is further refined after the bleaching.

13. Method according to one or more of the preceding Claims 7 to 12, wherein the material is punched to form compresses, wherein the cutting to size takes place before or after the bleaching and the compresses are consolidated at the edges, in particular by means of water jet needling.

14. Method according to Claim 13, wherein the compresses are sterilised and individually packaged.

15. Method according to one of the preceding claims, wherein the cotton fibres used in the formation of the nonwoven are unbleached or partially bleached.

## Revendications

1. Matériau de bandage composé d'un matériau non-tissé médical, comprenant un matériau plat dont les deux faces supérieures sont enchevêtrées en vue de former un principe actif en non-tissé, le matériau à principes actifs en non-tissé étant blanchi et le matériau non-tissé étant composé de fibres de coton ou d'un mélange de fibres de coton.

2. Matériau de bandage selon la revendication 1, dans lequel l'enchevêtrement s'effectue suivant le procédé Kunit et/ou Multiknit.

3. Matériau de bandage selon la revendication 1 ou 2, dans lequel les fibres entre les faces supérieures sont alignées en formant des espaces vides.

4. Matériau de bandage selon l'une des revendications précédentes, dans lequel le matériau à principes actifs en non-tissé peut être amélioré par le biais d'étapes d'amélioration du textile.

5. Matériau de bandage selon l'une des revendications précédentes, dans lequel des principes actifs médicaux ou cosmétiques sont fixés sur les fibres du matériau à principes actifs en non-tissé.

6. Matériau de bandage selon l'une des revendications précédentes, lequel est une compresse ou un tampon.

7. Procédé pour fabriquer un matériau non-tissé médical pour matériaux de bandage à partir de fibres de coton ou d'un mélange de fibres de coton selon l'une des revendications précédentes, dans lequel le matériau plat est enchevêtré au niveau des deux surfaces supérieures au moyen d'un procédé de tricotage en vue de former un matériau à principes actifs en non-tissé, et dans lequel le matériau à principes actifs en non-tissé est ensuite soumis à un processus de blanchiment.

8. Procédé selon la revendication 7, dans lequel le matériau à principes actifs en non-tissé est consolidé.

9. Procédé selon la revendication 7 ou 8, dans lequel la densité ou le poids par unité de surface du matériau à principes actifs en non-tissé est augmentée.

10. Procédé selon au moins l'une des revendications 7 à 9, dans lequel la consolidation ou bien l'augmentation du poids par unité de surface ou bien encore l'augmentation de la densité du matériau à principes actifs en non-tissé se fait par un processus de blanchiment.

11. Procédé selon au moins l'une des revendications 7 à 10, dans lequel le procédé de blanchiment est un procédé de blanchiment par rotation.

12. Procédé selon une ou plusieurs des revendications 7 à 11, dans lequel le matériau non-tissé est encore amélioré après le blanchiment.

13. Procédé selon une ou plusieurs des revendications 7 à 12, dans lequel le matériau est découpé en compresses, le conditionnement ayant lieu avant ou après le blanchiment et les compresses étant consolidées sur le bord, notamment au moyen d'un aiguilletage par jet d'eau.

14. Procédé selon la revendication 13, dans lequel les compresses sont stérilisées et emballées à l'unité.

15. Procédé selon l'une des revendications précédentes, dans lequel les fibres de coton employées ne sont pas blanchies ou sont partiellement blanchies lors de la formation du non-tissé.
